# EUROPEAN PATENT APPLICATION

(11) **EP 4 517 644 A1**
(43) Date of publication of application: **05.03.2025**
(21) Application number: 23194574.2
(22) Date of filing: 31.08.2023
(51) Int. Cl.: G06T 1/00, G06F 21/16, G16H 30/40

(54) **INJECTING COLORED NOISE IN MEDICAL IMAGES FOR PROTECTION AGAINST TAMPERING**

(71) Applicant: Koninklijke Philips N.V., 5656 AG Eindhoven (NL)
(72) Inventor: MEINEKE, Jan Jakob, Eindhoven (NL); SCHUELKE, Christophe Michael Jean, Eindhoven (NL)
(74) Representative: Philips Intellectual Property & Standards

(57) **Abstract**

The present invention relates to a computer-implemented method of protecting a medical image against tampering. The method comprises the generation of colored noise comprising a predefined statistical property, receiving a medical image, and augmenting the received medical image with the colored noise before providing the augmented medical image.

## Description

### FIELD OF THE INVENTION

The present invention relates to a computer-implemented method of protecting a medical image against tampering, a data processing apparatus, a computer program, and a computer-readable storage medium.

### BACKGROUND OF THE INVENTION

In particular in medical imaging, the images acquired on a specific hardware are typically exported, for example as a DICOM image, to a picture archiving and communication system PACS.

However, malicious attacks on hospital networks have been described in which neural networks have been used to modify data during transmission from scanner to PACS, introducing or removing lesions. In addition, storing and providing the images on a picture archiving and communication system enables in principle the possibility for any third party to implement post-processing software that applies denoising or other image enhancements, such as super-resolution to the images made available on the picture archiving and communication system. The application of digital signatures or watermarks to images is known but rarely used.

The inventors of the present invention have thus found that it would be advantageous to have an improved method of establishing authenticity of an image and/or verifying that the image cannot be manipulated or processed in any unauthorized way.

### SUMMARY OF THE INVENTION

It is an object of the present invention to provide an improved method of protecting an image against tampering and unauthorized processing.

The object of the present invention is solved by the subject matter of the independent claims, wherein further embodiments are incorporated in the dependent claims.

The described embodiments similarly pertain to the computer-implemented method of protecting a medical image against tampering, the data processing apparatus, the computer program, and the computer-readable storage medium. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a computer-implemented method of protecting a medical image against tampering. The method comprises the steps of receiving a medical image, generating colored noise, the colored noise comprising a predefined statistical property,
augmenting the medical image with the colored noise, and providing the augmented medical image.

Thus, methods and applications of purposefully introducing colored noise in medical images are described. Although it is easy to generate colored noise with chosen statistical properties, it may be harder to determine the specific noise characteristics and to remove such colored noise.

Denoising neural networks have recently shown great promise in medical imaging, surpassing the performance of classic image denoising. Neural networks for denoising images can be trained on images in a very generic fashion, and can be applied for denoising to any image or data that exhibits the same noise characteristics as was used during training of the neural network.

However, it is important that the image denoising using a trained neural network is applied on an image that exhibits the correct noise characteristics, e.g. complex independent Gaussian noise in the case of standard Cartesian magnetic resonance imaging. Thus, already the previous application of image correction steps such as geometry correction for treating gradient non-linearities in magnetic resonance imaging or frequency filters makes it harder to apply denoising neural networks, or any networks trained for further data processing like segmentation, for example. Nevertheless, the effects of these processing steps may be rather predictable and it may be possible to incorporate their effect in the training process of the neural network.

In contrast to that, the injection of additional specific noise with preferably variable noise characteristics has the potential to create a sufficiently large domain shift to render the application of neural networks useless, which are not trained to remove this specific noise characteristic. The noise characteristic may be variable per image, and/or may be varying across the image. Potential applications may include preventing third-party application of denoising neural networks, and ensuring authenticity of images in the face of malicious attacks.

Therefore, the presence of a correlated noise pattern in the image could be used to detect tampering. In particular, an attack on these images provided with colored noise would likely result in images with visible corruption or degradation, instead of images in which nothing raises suspicion of corruption, as intended by the attackers. In addition or alternatively, an attack on these images would probably alter the noise characteristics enough for the corruption to be detected automatically, for example, with a specifically trained neural network.

Thus, the augmentation of a medical image with noise with known statistical properties together with the capability of removing such noise with a suitably trained network may facilitate and increase the adoption of countermeasures in order to protect the image against tampering and unauthorized processing.

In addition, the protection against tampering is not immediately obvious to a third party, in contrast to, for example, the application of a watermark.

In an embodiment of the invention, the step of augmenting the medical image with the colored noise comprises adding the colored noise to the medical image.

In an embodiment of the invention, the step of augmenting the medical image with the colored noise comprises multiplying the colored noise to the medical image.

In an embodiment of the invention, the step of generating the colored noise comprises modulating a frequency distribution of the medical image.

In an embodiment of the invention, the frequency distribution is a spatial frequency distribution of the medical image.

In an embodiment of the invention, modulating the frequency distribution of the medical image comprises multiplying the medical image with a weighting function in the acquisition domain of the medical image.

In an embodiment of the invention, the step of generating the colored noise comprises using a random stochastic process.

In an embodiment of the invention, the random stochastic process comprises a Markov chain.

In an embodiment of the invention, the step of augmenting the medical image with the colored noise comprises augmenting the medical image with the colored noise in an image-space, in a k-space, on a coil-image-level of the medical image, or in a transform domain of the medical image. Thus, it may also be possible to apply the colored noise in an arbitrary transform domain, e.g. wavelet, or of any invertible, preferably unitary, transformation.

In an embodiment of the invention, the method comprises the step of removing measurement noise from the medical image before augmenting the medical image with the colored noise.

In an embodiment of the invention, the predefined statistical property of the colored noise is characterized by a noise power spectrum.

In an embodiment of the invention, the step of augmenting the medical image with the colored noise is performed on a sub-region of the medical image or on the complete medical image.

In an embodiment of the invention, the method comprises the step of removing the colored noise from the medical image augmented with the colored noise in order to provide the image for further processing. The added noise can be removed by utilizing a specifically trained neural network.

In an embodiment of the invention, the method comprises the step of analyzing a medical image previously augmented with colored noise with respect to a noise characteristic, thereby verifying an authenticity of the medical image.

According to another aspect of the invention, there is provided a data processing apparatus comprising means for carrying out the steps of the method according to any of the preceding embodiments.

According to another aspect of the invention, there is provided a computer program comprising instructions which, when the program is executed by a computer, cause the computer to carry out the steps of the method according to any of the preceding embodiments.

According to another aspect of the invention, there is provided a computer-readable storage medium comprising instructions which, when executed by a computer, cause the computer to carry out the steps of the method according to any of the preceding embodiments.
Thus, the benefits provided by any of the above aspects equally apply to all of the other aspects and vice versa.

In summary, the invention relates to a computer-implemented method of protecting a medical image against tampering. The method comprises the generation of colored noise comprising a predefined statistical property, receiving a medical image, and augmenting the received medical image with the colored noise before providing the augmented medical image.

One of the advantages of embodiments of the present invention may be that authenticity of an image can be established. Another advantage may reside in that a protected image cannot be manipulated or processed in an unauthorized way. Another advantage may reside in that an image can be protected against third party tampering.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a block diagram of a computer-implemented method of protecting a medical image against tampering according to an embodiment of the invention.
Fig. 2 shows a flow scheme of a computer-implemented method of protecting a medical image against tampering according to an embodiment of the invention.

### DETAILED DESCRIPTION OF EMBODIMENTS

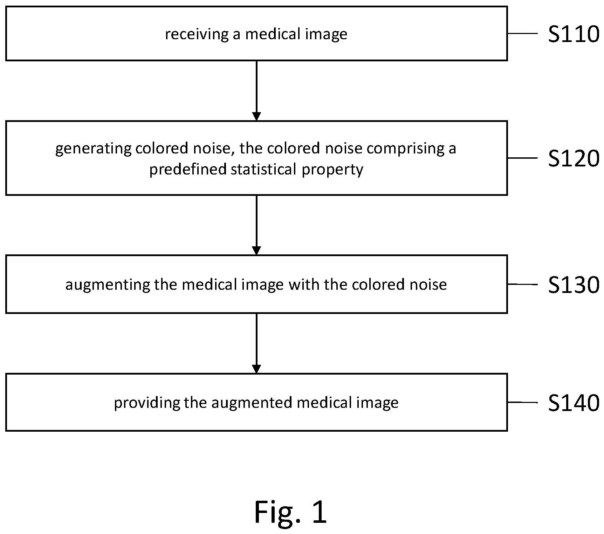

Fig. 1 shows a block diagram of a computer-implemented method of protecting a medical image 110 against tampering according to an embodiment of the invention. The method comprises step S 110 of receiving a medical image 110, and step S120 of generating colored noise 120, the colored noise 120 comprising a predefined statistical property. The method comprises further step S130 of augmenting the medical image 110 with the colored noise 120, and step S140 of providing the augmented medical image 111.

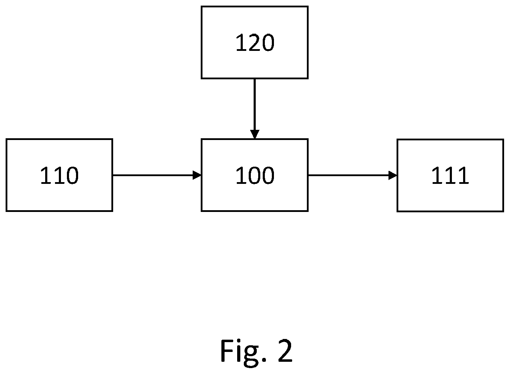

Fig. 2 shows a flow scheme of a computer-implemented method of protecting a medical image 110 against tampering according to an embodiment of the invention. A medical image 110 is received, which is preferably acquired by a medical imaging system for magnetic resonance imaging, computed tomography or ultra sound imaging. This is followed by the intentional injection of colored noise 120 into the image reconstruction chain at predefined point of the image reconstruction chain. The colored noise 12 can be injected, for example, by addition or by multiplication into the image. This can be done with or without first removing noise originating from uncorrelated complex Gaussian noise in the measurement domain. The resulting augmented medical image 111 with added colored noise 120 will yield similar visual appearance to the human eye as a medical image 110 that is not augmented with colored noise 120. However, it will be much harder, for example by using third-party denoising software that it is not trained to remove this specific type of noise, to remove this intentionally added colored noise 120 to the medical image 110. The augmentation of the medical image 110 with the colored noise 120 can be performed in a data processing apparatus 100 comprising means for carrying out the steps of the method according to the invention. The colored noise 120 can be generated preferably in the data processing apparatus 100 that augments the medical image 110 with the colored noise 120, but can also be generated in an external noise generator. With the augmentation of the medical image 110 with the colored noise 120, the medical image 110 is transformed into the augmented medical image 111 that can be provided or saved to a picture archiving and communication system.

At a suitable point in the image production or reconstruction chain, the colored noise 120 can be created by a random statistical process. Many methods are known to create colored noise. One may be to modulate a spatial frequency distribution of the image, for example by multiplying it with a weighting function in the acquisition domain, which will result in noise that is correlated between neighboring voxels in image space. Another method may be to use random stochastic processes, e.g. Markov chains, which can produce correlated samples.

The colored noise 120 can be added or multiplied to the medical image 110 in image-space, in k-space, on the coil-image-level, or it can be injected in any transformation domain of the medical image 110.

Preferably, the original noise resulting from the measurement in the medical imaging system can be removed or subtracted before adding the colored noise 120 to the image.

Preferably, colored noise can be added to a region of the image, comparable to a subtle watermark, which will make it possible to establish authenticity of the image and/or to verify whether the image has been tampered with. Automatic removal of the watermark of colored noise for display-only can be considered.

The colored noise added to the image cannot be easily subtracted by anyone who does not know the generating statistics of the colored noise. Images augmented with colored noise will appear to the human eye as if they were not denoised from measurement noise, so that they remain usable. However, full denoising capabilities are only available after having removed the additional colored noise injected into the image.

In case of authenticity verification of the image, the colored noise, or at least a watermark, can be added to parts of the image or across the whole image. It is important to realize that the watermark of the colored noise may not be deterministic and can therefore not be easily identified or located by outsiders or third-parties. Although it may be relatively easy to detect the presence of colored noise, it is not easy to know which exact noise power spectrum was used for its generation. However, for removing the colored noise, it is necessary to know the exact noise power spectrum used for its generation and to train a neural network to remove the respective noise. Having trained a suitable neural network it can be used to remove the colored noise from the augmented images again.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: data processing apparatus
- 110: medical image
- 111: augmented medical image
- 120: colored noise

## Claims

1. A computer-implemented method of protecting a medical image against tampering; the method comprising the steps of
receiving (S110) a medical image (110);
generating (S120) colored noise (120), the colored noise (120) comprising a predefined statistical property;
augmenting (S130) the medical image (110) with the colored noise (120); and
providing (S140) the augmented medical image (111).

2. The method according to claim 1, wherein the step of augmenting the medical image (110) with the colored noise (120) comprises adding the colored noise (120) to the medical image (110).

3. The method according to claim 1, wherein the step of augmenting the medical image (110) with the colored noise (120) comprises multiplying the colored noise (120) to the medical image (110).

4. The method according to any of the preceding claims, wherein the step of generating the colored noise (120) comprises modulating a frequency distribution of the medical image (110).

5. The method according to claim 4, wherein the frequency distribution is a spatial frequency distribution of the medical image (110).

6. The method according to any of claims 4 or 5, wherein modulating the frequency distribution of the medical image (110) comprises multiplying the medical image (110) with a weighting function in the acquisition domain of the medical image (110).

7. The method according to any of claim 1 to 3, wherein the step of generating the colored noise (120) comprises using a random stochastic process.

8. The method according to claim 7, wherein the random stochastic process comprises a Markov chain.

9. The method according to any of the preceding claims, wherein the step of augmenting the medical image (110) with the colored noise (120) comprises augmenting the medical image (110) with the colored noise (120) in an image-space, in a k-space, on a coil-image-level of the medical image, or in a transform domain of the medical image.

10. The method according to any of the preceding claims, wherein the method comprises the step of removing measurement noise from the medical image (110) before augmenting the medical image (110) with the colored noise (120).

11. The method according to any of the preceding claims, wherein the predefined statistical property of the colored noise (120) is **characterized by** a noise power spectrum.

12. The method according to any of the preceding claims, wherein the step of augmenting the medical image (110) with the colored noise (120) is performed on a sub-region of the medical image (110) or on the complete medical image (110).

13. A data processing apparatus (100) comprising means for carrying out the steps of the method according to any of claims 1 to 12.

14. A computer program comprising instructions which, when the program is executed by a computer, cause the computer to carry out the steps of the method according to any of claims 1 to 12.

15. A computer-readable storage medium comprising instructions which, when executed by a computer, cause the computer to carry out the steps of the method according to any of claims 1 to 12.
